# FASCICULE DE BREVET EUROPEEN

(11) **EP 0 711 534 B1**
(45) Date de publication et mention de la délivrance du brevet: **19.01.2000**
(21) Numéro de dépôt: 95402198.6
(22) Date de dépôt: 29.09.1995
(51) Int. Cl.: A61F 2/36, A61F 2/30

(54) **Tige fémorale pour prothèse de hanche**
Femurschaft für Hüftprothese
Femoral stem for prosthesis

(30) Priorité: 12.10.1994 FR 9412177
(43) Date de publication de la demande: 15.05.1996
(73) Titulaire: Prost, Didier, Dr., 13009 Marseille (FR); Cermolacce, Christophe, Dr., 13012 Marseille (FR)
(72) Inventeur: Prost, Didier, Dr., 13009 Marseille (FR); Cermolacce, Christophe, Dr., 13012 Marseille (FR)
(74) Mandataire: Domange, Maxime

(56) Documents cités:
- EP-A- 0 095 440
- EP-A- 0 363 151
- WO-A-91/03992
- WO-A-94/12122
- FR-A- 2 661 328
- US-A- 4 718 909

## Description

La présente invention concerne une tige fémorale pour prothèse de hanche à pose sans ciment.

On connaît déjà du document WO-A-9 412 122 une tige fémorale comportant une partie proximale élargie servant d'appui métaphysaire dans le fémur et une partie distale effilée à insérer dans le canal médullaire du fémur et équipée à son extrémité distale d'un bouchon de centrage en matériau résorbable.

Ce type de bouchon coiffant l'extrémité distale de la tige présente l'avantage, d'une part, de stabiliser l'implant les premiers mois pour permettre sa réhabitation osseuse, et d'autre part, d'éviter le passage des contraintes en bout de tige après résorption de ce bouchon, d'éliminer ainsi l'ostéolyse périprothétique et en conséquence d'éviter l'apparition de douleurs persistantes au niveau de la cuisse où est implantée la tige fémorale, qui résulteraient autrement des contraintes engendrées entre un bouchon non résorbable et le canal médullaire du fémur.

Toutefois, une réaction inflammatoire locale non bactérienne apparaît généralement 2 à 4 mois après l'opération de pose de la tige fémorale pourvue d'un bouchon résorbable et résulte en un écoulement fistulaire vers la surface de la cuisse.

Par ailleurs, les tiges fémorales actuellement connues ne permettent pas un calage et un centrage optima de la tige fémorale dans le fémur.

La présente invention a donc pour but d'éliminer les inconvénients précités et de proposer une tige fémorale pour prothèse de hanche qui assure un calage et un centrage optimal de la tige dans le fémur.

A cet effet, la présente invention a pour objet une tige fémorale pour prothèse de hanche, comprenant une partie proximale élargie servant d'appui métaphysaire dans le fémur et une partie distale généralement effilée à 5 5 insérer dans le canal médullaire du fémur et équipée d'un élément de centrage distal en matériau biorésorbable, caractérisée en ce que ledit élément de centrage distal est une rondelle munie d'un perçage central pour recevoir ladite partie distale effilée et en ce que la tige fémorale comporte une protubérance de calage métaphysaire en saillie de la face dite postérieure de la tige et destinée à venir en appui interne contre l'endocorticale fémorale postérieure naissante sous le petit trochanter.

En prévoyant une rondelle en matériau biorésorbable, au lieu d'un bouchon s'adaptant à l'extrémité distale de la tige fémorale, on réduit le risque de formation éventuelle de granulome inflammatoire ou de pseudo-tumeur en réaction à l'introduction d'un corps étranger, par diminution notamment du volume de matériau résorbable implanté.

En outre, la rondelle précitée coopère avec la protubérance de calage métaphysaire pour assurer un centrage de la partie distale de la tige et un calage de la partie proximale de celle-ci optima.

En effet, les tiges fémorales actuellement connues comportent uniquement une bosse au niveau de la face dite externe de la tige pour venir en appui interne contre l'endocorticale externe du massif trochantérien fémoral, ce qui permet d'assurer un calage dans la direction interne-externe de la tige, mais non dans la direction antero-postérieure.

En particulier, la protubérance dans l'invention coopère avec la rondelle pour éviter un mouvement notamment rotatoire de la tige dans le canal médullaire et un contact inopportun de l'extrémité distale de la tige avec la corticale fémorale.

Selon une autre caractéristique de l'invention, en vue sagittale de la tige, la protubérance précitée présente un rayon de courbure égal à 1/3 ± 1/16 du rayon de courbure de la face postérieure de la tige ou 1/2 ± 1/16 du rayon de courbure de la face dite antérieure de la tige.

Selon encore une autre caractéristique de l'invention, en section transversale de la tige, sensiblement à la hauteur du sommet de la protubérance, le rayon de courbure de celle-ci est égal à 1/2 ± 1/16 du rayon de courbure de la face antérieure de la tige.

On peut prévoir par exemple que la protubérance précitée s'étende sur la face postérieure de la tige, en partant de l'extrémité proximale de celle-ci, entre une valeur comprise entre 1/6 et 1/5 de la hauteur de la tige et une valeur comprise entre 2/5 et 1/2 de la hauteur de la tige.

Selon encore une autre caractéristique de l'invention, la protubérance précitée est circonscrite par un angle au centre de courbure de la face antérieure de la tige, compris entre 8° et 12°.

D'autre part, le perçage central de la rondelle peut être conformé de sorte qu'elle soit située à une hauteur de 15 ± 2 mm de l'extrémité distale de la tige.

Par exemple, le perçage central peut être tronconique et présenter un angle d'ouverture égal à 15 ± 2°.

Par ailleurs, on peut prévoir que l'épaisseur de la rondelle soit égale à 5 ± 1 mm.

Le diamètre externe de la rondelle est généralement compris entre 12 et 32 mm, suivant la taille du canal médullaire du fémur dans lequel la tige fémorale doit être implantée.

Avantageusement, la rondelle présente au moins un évidement sur sa périphérie, de préférence trois évidements régulièrement espacés, pour diminuer le volume implanté et les surfaces de contact entre la rondelle et les corticales fémorales.

L'invention sera mieux comprise et d'autres buts, caractéristiques, détails et avantages de celle-ci apparaîtront plus clairement au cours de la description explicative qui va suivre d'un mode de réalisation particulier, actuellement préféré de l'invention, donné uniquement à titre d'exemple illustratif et non limitatif, en référence aux dessins schématiques annexés, dans lesquels.

La figure 1 est une vue en élévation et de profil de la tige fémorale de l'invention, suivant la flèche I de la figure 2, montrant le bord dit externe de la tige.

La figure 2 est une vue en élévation postérieure de la tige fémorale de la figure 1, suivant la flèche II, montrant la face dite postérieure de la tige.

La figure 3 est une vue en coupe transversale de la tige de la figure 2, suivant la ligne III-III.

La figure 4 est une vue de dessous de la rondelle en matériau biorésorbable illustrée sur la figure 5, destinée à être enfilée sur la partie distale effilée d'une tige fémorale de l'invention.

La figure 5 est une vue en coupe de la rondelle de la figure 4, suivant la ligne V-V.

La figure 6 est une vue analogue à la figure 1, mais représentant la tige fémorale de l'invention équipée d'une rondelle en matériau biorésorbable et d'un col prothétique surmonté d'une tête fémorale.

La figure 7 est une vue en élévation postérieure et partiellement arrachée de la tige fémorale de la figure 6, suivant la flèche VII, représentant un col modulaire prothétique.

La figure 8 est une vue schématique et en plan d'un col modulaire prothétique coudé.

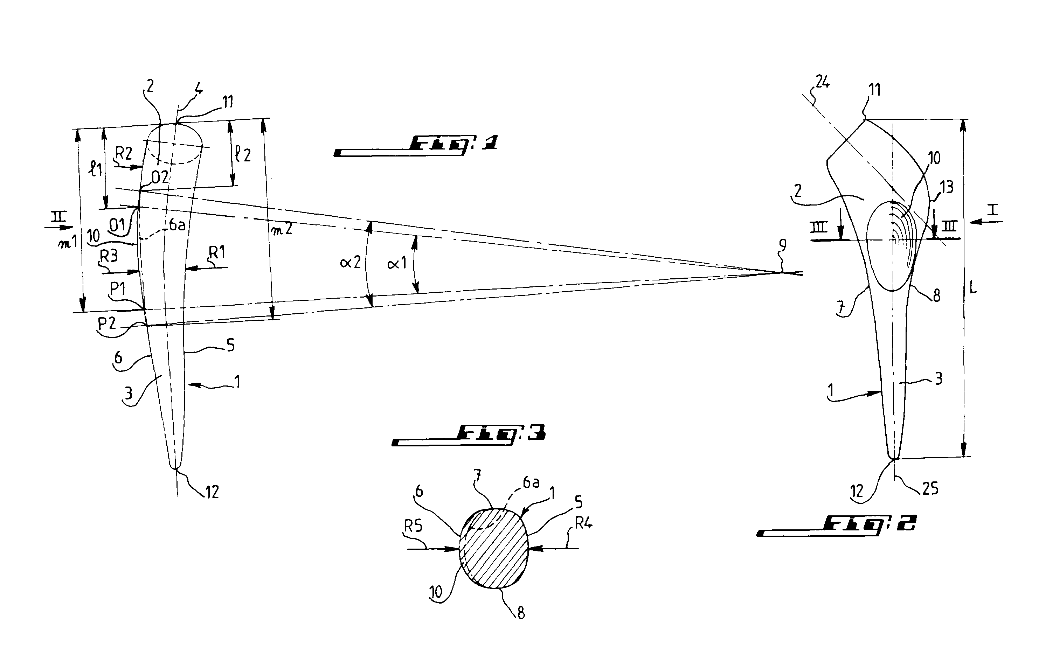

Suivant l'exemple de réalisation représenté sur les figures 1 à 3, la tige fémorale 1 de l'invention comprend une partie proximale élargie 2 et une partie distale généralement effilée 3.

En vue sagittale de la tige 1, c'est-à-dire en vue de profil postéro-antérieur (de la gauche vers la droite sur la figure 1), la tige fémorale 1 présente une courbure le long de son axe anatomique 4.

Sur la vue sagittale de la figure 1, la tige 1 présente une face dite antérieure concave 5 et une face dite postérieure convexe 6.

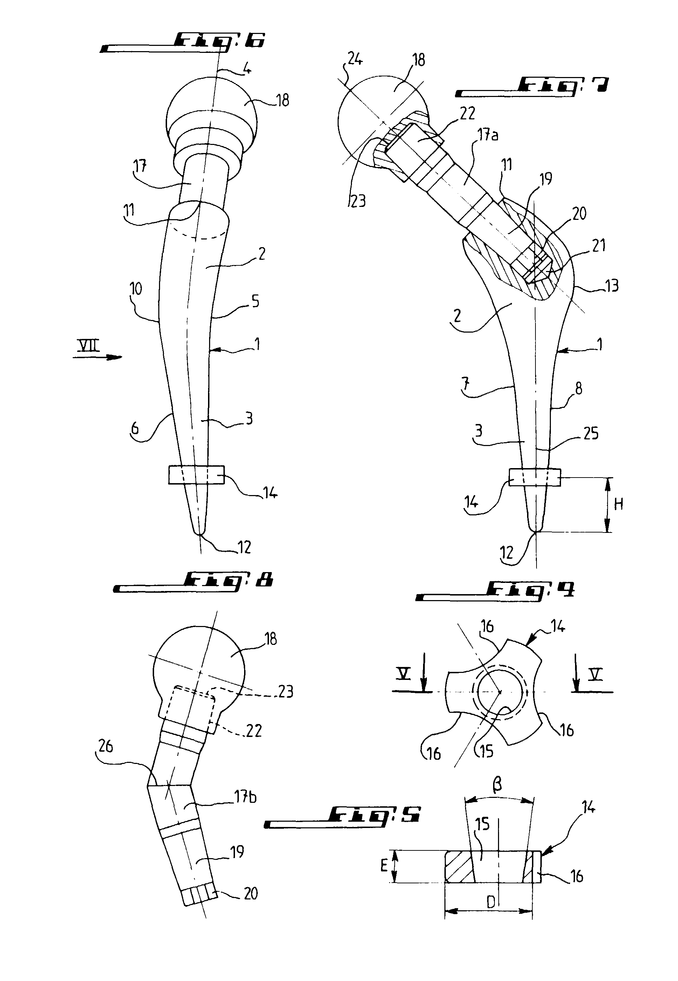

En vue postérieure, c'est-à-dire sur la figure 2, la tige fémorale 1 comporte un bord dit interne 7 et un bord dit externe 8.

Au sens de la présente invention, les faces antérieure 5, postérieure 6, et les bords interne 7 et externe 8 correspondent à l'orientation de la tige lorsqu'elle est implantée dans un fémur d'un individu.

On voit sur la figure 1 que la face antérieure concave 5 présente un centre de courbure 9 associé à un rayon de courbure R1 indiqué par une flèche.

On a représenté par des traits mixtes 6a sur la figure 1 la courbure de la face postérieure convexe 6 que pourrait adopter une tige classique, le rayon de courbure R2 indiqué par une flèche étant associé à cette courbure.

La tige fémorale 1 de l'invention comporte une protubérance 10 sur la face postérieure convexe 6, le rayon de courbure R3 indiqué par une flèche étant associé à cette protubérance 10.

La protubérance 10 vient s'appuyer sur la corticale osseuse naissante du fémur, au niveau du petit trochanter.

Cet appui cortical permet un meilleur blocage de la partie proximale 2 de la prothèse et améliore la transmission des contraintes à l'os, ce qui augmente la longévité de la prothèse et réduit l'ostéolyse périprothétique.

La protubérance 10 est circonscrite sur la figure 1 par un angle au centre 9 compris entre α1 = 8° et α2 = 11,5°.

En partant de l'extrémité proximale 11 de la tige 1, la protubérance 10 s'étend sur la hauteur de la tige, à partir d'un point O1 ou O2 jusqu'à un point P1 ou P2, selon que la protubérance 10 est circonscrite par l'angle α1 ou α2 respectivement.

Dans le sens de la hauteur de la tige 1, les points O1 et O2 sont distants de l'extrémité proximale 11 d'une distance ℓ1 = 1/5L et ℓ2 = 1/6L, respectivement (voir figure 1), où L représente la hauteur totale de la tige 1 (voir figure 2).

Les points P1 et P2 sont distants de l'extrémité proximale 11 d'une distance m1 = 2/5L et m2 = 1/2L, respectivement.

Les valeurs de ℓ1, ℓ2, m1 et m2 peuvent varier de ± 15% sans sortir du principe de l'invention.

Les valeurs des rayons de courbure R1, R2 et R3 peuvent être égales à 200 mm, 300 mm et 100 mm respectivement.

On voit sur la figure 2 que la hauteur L qui est définie entre l'extrémité proximale 11 et l'extrémité distale 12 de la tige 1, peut être comprise par exemple entre 100 et 150 mm.

De la même manière que sur la figure 1, on a indiqué en traits mixtes 6a sur la figure 3 la courbure que pourrait adopter une tige classique sans la protubérance de l'invention.

En section transversale sur la figure 3, la face antérieure 5 de la tige est convexe et présente un rayon de courbure R4 indiqué par une flèche.

Toujours sur la figure 3, la protubérance 10 présente en section transversale un rayon de courbure R5 qui peut être égal à un 1/2 ± 1/16 de R4, avec R4 variant en fonction des tailles.

On voit sur la figure 2, au niveau de la partie élargie proximale 2 de la tige 1, une bosse 13 qui fait saillie du bord externe 8 et qui est destinée à venir en appui interne contre le grand trochanter du fémur.

La section transversale représentée sur la figure 3 passe sensiblement par le sommet de la protubérance 10, c'est-à-dire à environ 1/3 ± 1/16 L, en partant de l'extrémité proximale 11 de la tige 1.

On a représenté sur les figures 4 et 5 une rondelle en matériau biorésorbable 14 qui est destinée à être enfilée sur la partie distale effilée 3 de la tige 1 (voir figures 6 et 7).

La rondelle 14 peut présenter une épaisseur E égale sensiblement à 5 ± 1 mm et comporte un perçage central tronconique 15 pour recevoir la partie effilée distale 3 précitée.

Le perçage central 15 présente un angle d'ouverture β égal sensiblement à 15 ± 2°.

Dans l'exemple de réalisation particulier illustré sur les figures 4 et 5, la rondelle 14 présente trois évidements 16, régulièrement espacés sur la surface latérale circonférentielle de la rondelle 14, de manière à réduire le volume implanté et les zones de contact entre la rondelle 14 et les corticales fémorales.

On peut prévoir par exemple que pour un diamètre de rondelle 14 égal à 12 mm, le plus grand diamètre interne du perçage central 15 soit égal à 7 mm et que les évidements 16 présentent, dans le plan de la rondelle, un rayon de courbure de 10 mm.

On a défini sur la figure 5 une distance D séparant un point du pourtour de la rondelle 14 du point le plus interne d'un évidement 16, le long d'un diamètre de la rondelle.

Pour un diamètre de rondelle 14 égal à 12, 14, 16, 18, 20, 24, 28 et 32 mm, on peut définir une distance D égale respectivement à 10, 10, 13, 14, 15, 17, 19 et 21 mm.

Bien entendu, le nombre et la forme des évidements 16 peuvent être modifiés de manière quelconque.

Les dimensions et l'angle d'ouverture du perçage central 15 de la rondelle 14 sont déterminés de sorte que la rondelle 14 soit située approximativement à une distance H égale à 15 ± 2 mm de l'extrémité distale 12 de la tige 1 (voir figure 7).

On voit sur la figure 6 que la tige 1 est équipée à son extrémité proximale 11 d'un col prothétique 17 surmonté d'une tête sphérique fémorale 18.

Le col prothétique 17 et/ou la tête fémorale 18 peuvent être prévus monobloc avec la tige 1, ou en pièces séparées comme indiqué sur la figure 7.

On a représenté sur la figure 7 un col modulaire prothétique 17a dont les extrémités longitudinales sont du type à cône "morse".

Le cône inférieur 19 présente une partie cannelée ou à section polygonale 20 qui s'adapte dans un alésage correspondant 21 dans la partie proximale 2 de la tige 1, à partir de l'extrémité proximale 11 de celle-ci.

La fixation par cône morse produit un effet de serrage généralement connu.

Le cône supérieur 22 s'emboîte dans un alésage correspondant 23 pratiqué dans la tête fémorale 18.

Le col modulaire 17a restitue à la fois l'inclinaison anatomique 24 et la longueur de la tête fémorale par rapport à la verticale 25.

On a représenté sur la figure 8 une variante de réalisation du col modulaire 17b qui comporte un coude 26 sensiblement en son milieu, ce qui permet d'adapter l'inclinaison et la position du col modulaire en fonction des différents types de hanche rencontrés par le chirurgien.

Bien que l'invention ait été décrite en liaison avec un mode de réalisation particulier, il est évident qu'elle n'y est nullement limitée et qu'on peut lui apporter de nombreuses variantes et modifications sans pour autant sortir de son cadre tel que défini dans les revendications.

## Revendications

1. Tige fémorale (1) pour prothèse de hanche, comprenant une partie proximale élargie (2) servant d'appui métaphysaire dans le fémur et une partie distale généralement effilée (3) à insérer dans le canal médullaire du fémur et équipée d'un élément de centrage distal en matériau biorésorbable (14), caractérisée en ce que ledit élément de centrage distal est une rondelle (14) munie d'un perçage central (15) pour recevoir ladite partie distale effilée (3) et en ce que la tige fémorale (1) comporte une protubérance de calage métaphysaire (10) en saillie de la face dite postérieure (6) de la tige (1) et destinée à venir en appui interne contre l'endocorticale fémorale postérieure naissante sous le petit trochanter.

2. Tige fémorale selon la revendication 1, caractérisée en ce qu'en vue sagittale de la tige, la protubérance (10) précitée présente un rayon de courbure (R3) égal à 1/3 ± 1/16 du rayon de courbure (R2) de la face postérieure (6) de la tige ou 1/2 ± 1/16 du rayon de courbure (R1) de la face dite antérieure (5) de la tige.

3. Tige fémorale selon la revendication 1 ou 2, caractérisée en ce qu'en section transversale de la tige, sensiblement à la hauteur du sommet de la protubérance (10), le rayon de courbure (R5) de celle-ci est égal à 1/2 ± 1/16 du rayon de courbure (R4) de la face antérieure (5) de la tige.

4. Tige fémorale selon l'une quelconque des revendications précédentes, caractérisée en ce que la protubérance (10) précitée s'étend sur la face postérieure (6) de la tige (1), en partant de l'extrémité proximale (11) de celle-ci, entre une valeur (ℓ1, ℓ2) comprise entre 1/6 et 1/5 de la hauteur (L) de la tige et une valeur (m1, m2) comprise entre 2/5 et 1/2 de la hauteur (L) de la tige.

5. Tige fémorale selon l'une quelconque des revendications précédentes, caractérisée en ce que la protubérance précitée (10) est circonscrite par un angle (α1, α2) au centre de courbure (9) de la face antérieure (5) de la tige, compris entre 8° et 12°.

6. Tige fémorale selon l'une quelconque des revendications précédentes, caractérisée en ce que le perçage central (15) de la rondelle (14) est conformé de sorte qu'elle soit située à une hauteur (H) de 15 ± 2 mm de l'extrémité distale (12) de la tige.

7. Tige fémorale selon l'une quelconque des revendications précédentes, caractérisée en ce que le perçage central (15) est tronconique et présente un angle d'ouverture (β) égal à 15 ± 2°.

8. Tige fémorale selon l'une quelconque des revendications précédentes, caractérisée en ce que l'épaisseur (E) de la rondelle (14) est égale à 5 ± 1 mm.

9. Tige fémorale selon l'une quelconque des revendications précédentes, caractérisée en ce que le diamètre externe de la rondelle (14) est compris entre 12 et 32 mm.

10. Tige fémorale selon l'une quelconque des revendications précédentes, caractérisée en ce que la rondelle (14) présente au moins un évidement (16) sur sa périphérie, de préférence trois évidements régulièrement espacés.

## Patentansprüche

1. Oberschenkelknochenschaft (1) für eine Hüftprothese, mit einem vergrößerten proximalen Teil (2), der in dem Oberschenkelknochen als Körperabstützung dient, und einem im Ganzen sich verjüngenden distalen Teil (3), der in den Markkanal des Oberschenkelknochens einzuführen und mit einem distalen Zentrierelement aus biologisch resorbierbarem Material (14) ausgestattet ist, dadurch gekennzeichnet, daß das distale Zentrierelement eine Scheibe (14) ist, die mit einer mittigen Bohrung (15) versehen ist, um den distalen, sich verjüngenden Teil (3) aufzunehmen, und daß der Oberschenkelknochenschaft (1) eine Ausbauchung (10) zur Körperpositionierung umfaßt, die aus der als rückseitige Fläche bezeichneten Fläche (6) des Schafts (1) heraussteht und dazu bestimmt ist, in innere Abstützung gegen die unter dem kleinen Rollhügel herausstehende hintere Endokortikale des Oberschenkelknochens zu gelangen.

2. Oberschenkelknochenschaft nach Anspruch 1, dadurch gekennzeichnet, daß die vorgenannte Ausbauchung (10) in Sagittalansicht des Schafts einen Krümmungsradius (R3) aufweist, der gleich 1/3 ± 1/16 des Krümmungsradius (R2) der rückseitigen Fläche (6) des Schafts oder 1/2 ± 1/16 des Krümmungsradius (R1) der als vordere Fläche bezeichneten Fläche (5) des Schafts ist.

3. Oberschenkelknochenschaft nach Anspruch 1 oder 2, dadurch gekennzeichnet, daß im Querschnitt des Schafts im wesentlichen auf der Höhe der höchsten Stelle der Ausbauchung (10) deren Krümmungsradius (R5) gleich 1/2 ± 1/16 des Krümmungsradius (R4) der vorderen Fläche (5) des Schafts ist.

4. Oberschenkelknochenschaft nach einem der vorhergehenden Ansprüche, dadurch gekennzeichnet, daß sich die vorgenannte Ausbauchung (10) auf der rückseitigen Fläche (6) des Schafts (1), ausgehend von dem proximalen Ende (11) desselben, zwischen einem Wert (11, 12) zwischen 1/6 und 1/5 der Höhe (L) des Schafts und einem Wert (ml, m2) zwischen 2/5 und 1/2 der Höhe (L) des Schafts erstreckt.

5. Oberschenkelknochenschaft nach einem der vorhergehenden Ansprüche, dadurch gekennzeichnet, daß die vorgenannte Ausbauchung (10) in einem Winkel (α1, α2) im Zentrum der Krümmung (9) der vorderen Fläche (5) des Schafts von zwischen 8° und 12° eingeschlossen ist.

6. Oberschenkelknochenschaft nach einem der vorhergehenden Ansprüche, dadurch gekennzeichnet, daß die mittige Bohrung (15) der Scheibe (14) so gestaltet ist, daß sie auf einer Höhe (H) von 15 ± 2 mm vom distalen Ende (12) des Schafts sitzt.

7. Oberschenkelknochenschaft nach einem der vorhergehenden Ansprüche, dadurch gekennzeichnet, daß die mittige Bohrung (15) kegelstumpfförmig ist und einen Öffnungswinkel (β) von 15 ± 2° hat.

8. Oberschenkelknochenschaft nach einem der vorhergehenden Ansprüche, dadurch gekennzeichnet, daß die Dicke (E der Scheibe (14) gleich 5 ± 1 mm ist.

9. Oberschenkelknochenschaft nach einem der vorhergehenden Ansprüche, dadurch gekennzeichnet, daß der äußere Durchmesser der Scheibe (14) zwischen 12 und 32 mm liegt.

10. Oberschenkelknochenschaft nach einem der vorhergehenden Ansprüche, dadurch gekennzeichnet, daß die Scheibe (14) auf ihrem Umfang mindestens eine Aussparung (16), vorzugsweise drei regelmäßig voneinander beabstandete Aussparungen, aufweist.

## Claims

1. A femoral rod (1) for a hip prosthesis, the rod comprising an enlarged proximal portion (2) serving as a metaphysis bearing member in the femur, and a generally tapering distal portion (3) for insertion in the medulary channel of the femur and fitted with a distal centering element of bioresorbable material (14), the rod being characterized in that said distal centering element is a washer (14) provided with a central hole (15) for receiving said tapering distal portion (3), and in that the femoral rod (1) has a metaphysis wedging protuberance (10) projecting from the "posterior" face (6) of the rod (1) and designed to bear internally against the posterior femoral endocortical beginning beneath the small trochanter.

2. A femoral rod according to claim 1, characterized in that in sagittal view of the rod, the above-mentioned protuberance (10) has a radius of curvature (R3) equal to 1/3 ± 1/16 the radius of curvature (R2) of the posterior face (6) of the rod, or equal to 1/2 ± 1/16 of the radius of curvature (R1) of the "anterior" face (5) of the rod.

3. A femoral rod according to claim 1 or 2, characterized in that, at the cross-section of the rod substantially level with the top of the protuberance (10), the radius of curvature (R5) of the protuberance is equal to 1/2 ± 1/16 of the radius of curvature (R4) of the anterior face (5) of the rod.

4. A femoral rod according to any preceding claim, characterized in that the above-mentioned protuberance (10) extends over the posterior face (6) of the rod (1), starting from the proximal end (11) thereof, between a value (λ1, λ2) lying in the range 1/6 to 1/5 of the height (L) of the rod, and a value (m1, m2) lying between 2/5 and 1/2 of the height (L) of the rod.

5. A femoral rod according to any preceding claim, characterized in the above-mentioned protuberance (10) is circumscribed by an angle (α1, α2) at the center of curvature (9) of the anterior face (5) of the rod lying in the range 8° to 12°.

6. A femoral rod according to any preceding claim, characterized in that the central hole (15) of the washer (14) is shaped so that it is situated at a height (H) of 15 mm ± 2 mm from the distal end (12) of the rod.

7. A femoral rod according to any preceding claim, characterized in that the central hole (15) is frustoconical and has a cone angle (β) equal to 15° ± 2°.

8. A femoral rod according to any preceding claim, characterized in that the thickness (E) of the washer (14) is equal to 5 mm ± 1 mm.

9. A femoral rod according to any preceding claim, characterized in that the outside diameter of the washer (14) lies in the range 12 mm to 32 mm.

10. A femoral rod according to any preceding claim, characterized in that the washer (14) has at least one recess (16) on its periphery, and preferably has three regularly spaced-apart recesses.
